# EUROPEAN PATENT APPLICATION

(11) **EP 4 847 008 A1**
(43) Date of publication of application: **02.12.2026**
(21) Application number: 25744479.4
(22) Date of filing: 14.01.2025
(51) Int. Cl.: A61K 8/44, A61Q 7/00, A61Q 5/00, A61P 17/14, A23K 20/142, A23L 33/175, A61K 31/198

(54) **COMPOSITION FOR PREVENTING HAIR LOSS, PROMOTING HAIR GROWTH OR INCREASING HAIR DENSITY**

(30) Priority: 24.01.2024 CN 202410097008
(71) Applicant: Suzhou Oulit Biopharm Co., Ltd., Suzhou, Jiangsu 215411 (CN)
(72) Inventor: ZHANG, Jian, Suzhou, Jiangsu 215122 (CN)
(74) Representative: Winger
(86) International application number: PCT/CN2025/072243
(87) International publication number: WO 2025/157036

(57) **Abstract**

The use of a composition comprising a N-long-chain acylamino acid and/or a salt thereof in preventing hair loss and/or promoting hair growth and/or increasing hair density. Particularly, the present invention relates to a related composition comprising a N-long-chain acylamino acid and a basic amino acid.

## Description

### TECHNICAL FIELD

The present disclosure relates to use of a specific composition in preventing hair loss and/or promoting hair growth and/or increasing hair density, and in particular, to a related composition comprising a N-long-chain acyl amino acid and/or a salt thereof.

### BACKGROUND

With the accelerating pace of life and increasing work intensity, hair loss diseases such as androgenetic alopecia (seborrheic alopecia) and alopecia areata are continuously increasing and becoming more common in younger populations. Such hair loss marres one's appearance and is unsightly. For the treatment of hair loss, the clinically commonly used western drugs are minoxidil and finasteride (among which finasteride can inhibit 5α-reductase *in vivo*, thereby inhibiting the production of dihydrotestosterone). However, the hair loss is prone to recurrence after discontinuation of either of the two drugs, and the two drugs may also cause side effects such as skin irritation, scalp pruritus, hirsutism on the face and hands, dizziness, anorexia, and tachycardia.

In addition to pharmaceutical treatments, the application of cosmetics to achieve the effects of preventing hair loss and/or promoting hair growth and/or increasing hair density has now been investigated. At present, commonly used options include various plant extracts, such as Platycladus orientalis, Polygonum multiflorum, Ligustrum lucidum, Ligusticum chuanxiong, Cuscuta chinensis, Gleditsia sinensis, Zingiber officinale, Eclipta prostrata, and Phryma leptostachya. It has also been reported that arginine, 2-substituted ketoamides, peptide-copper complexes, ferric compounds, pyrimidine derivatives, and the like have the effect of preventing hair loss and/or promoting hair growth.

For L-arginine, it has been reported for its property of increasing nitric oxide levels in the body; the body needs L-arginine and produces nitrogen from it. Nitric oxide has the effect of increasing blood flow, relaxing blood vessels, and stimulating hair growth.

WO1999013717A1 discloses a method for promoting hair growth, which comprises delivering a nitric oxide-releasing substance selected from L-arginine, L-arginine salts, and L-arginine derivatives by topical administration to a selected area of skin in need of hair growth. Embodiments thereof disclose the application of a penetrating cream containing L-arginine hydrochloride (15% w/v) and sodium chloride (10% w/v) to a bald area. The cream is applied to the bald area before bedtime every night and rubbed in thoroughly for maximum absorption. However, its actual efficacy is still limited.

CN101596152A discloses a combination of arginine and centella extract; EP1040815A1 discloses a combination of L-arginine, ethanol, and propylene glycol; US6019976A discloses a combination of sawtooth leaf extract, vitamin B6, vitamin B3, a zinc salt, and 10-15 wt% L-arginine; DE20315174U1 discloses a shampoo comprising arginine. These all intend to enhance the capability of inducing hair growth.

US6136860A discloses use of L-lysine in the manufacture of a medicament for preventing and treating hair loss in humans, but it is in an oral form.

Although various anti-hair loss agents and hair growth agents have been disclosed in the prior art, in actual use, effective substances such as L-arginine often cannot be effectively delivered into hair follicles, and the actual effect is very limited; or the related side effects are relatively large; or it is inconvenient to use, and some even need to follow medical instructions.

### SUMMARY

The present disclosure aims to provide a composition that can be used to prevent hair loss and/or promote hair growth and/or increase hair density, which exhibits significant effects and is also very safe, has no side effects, can be simply applied during the process of bathing and washing hair, and is convenient for consumers to use.

Specifically, the present disclosure discloses the following schemes.
[1]. Use of a composition comprising an N-long-chain acyl amino acid and/or a salt thereof in preventing hair loss and/or promoting hair growth and/or increasing hair density.
[2]. The use according to [1], characterized in that the use is cosmetic use.
[3]. The use according to [1], characterized in that the composition is a composition for topical administration.
[4]. The use according to any one of [1]-[3], characterized in that the composition comprises an N-long-chain acyl amino acid and a basic amino acid, or comprises a salt formed from the N-long-chain acyl amino acid and the basic amino acid, or comprises a sodium salt, a potassium salt, or an ammonium salt (e.g., a TEA salt) of the N-long-chain acyl amino acid.
[5]. The use according to any one of [1]-[4], characterized in that the composition further comprises an N-long-chain acyl amino acid dipeptide and/or a salt thereof.
[6]. The use according to any one of [1]-[5], characterized in that the composition further comprises an N-long-chain acyl amino acid dipeptide, or a salt formed from the N-long-chain acyl amino acid dipeptide and a basic amino acid, or a sodium salt, a potassium salt, or an ammonium salt (e.g., a TEA salt) of the N-long-chain acyl amino acid dipeptide.
[7]. The use according to any one of [1]-[6], characterized in that the N-long-chain acyl in the N-long-chain acyl amino acid is derived from a saturated or unsaturated linear or branched fatty acid with 8-22 carbon atoms; and/or
   the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, and (methyl)taurine.
[8]. The use according to any one of [1]-[7], characterized in that the N-long-chain acyl in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl; and/or
   the amino acid in the N-long-chain acyl amino acid is derived from one or more of alanine, glycine, glutamic acid, sarcosine, arginine, lysine, and (methyl)taurine, preferably alanine, and most preferably L-alanine.
[9]. The use according to any one of [4]-[8], characterized in that the basic amino acid is selected from one or more of arginine, lysine, citrulline, ornithine, creatine, histidine, diaminobutyric acid, and diaminopropionic acid, preferably arginine and/or lysine, and most preferably L-arginine.
[10]. The use according to any one of [5]-[9], characterized in that the N-long-chain acyl in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl; and/or
   the amino acid in the N-long-chain acyl amino acid dipeptide is derived from one or more of alanine, glycine, glutamic acid, sarcosine, arginine, lysine, and (methyl)taurine, preferably alanine, and most preferably L-alanine.
[11]. The use according to any one of [5]-[10], characterized in that the N-long-chain acyl amino acid dipeptide is selected from one or more of N-long-chain acyl glycyl glycine, N-long-chain acyl glutamyl glutamic acid, N-long-chain acyl alanyl alanine, and N-long-chain acyl sarcosyl sarcosine, preferably N-long-chain acyl alanyl alanine, and most preferably N-lauroyl-L-alanyl-L-alanine.
[12]. The use according to any one of [4]-[11], characterized in that the basic amino acid is present in an amount fully or partially neutralizing the N-long-chain acyl amino acid, preferably with the degree of neutralization being 85% or greater and less than 100%, more preferably 88% or greater and 96% or less; or
   the basic amino acid is present in an amount fully or partially neutralizing the N-long-chain acyl amino acid or the N-long-chain acyl amino acid dipeptide, preferably with the degree of neutralization being 85% or greater and less than 100%, more preferably 88% or greater and 96% or less.
[13]. The use according to any one of [1]-[12], characterized in that the N-long-chain acyl amino acid is lauroyl alanine (N-lauroyl-L-alanine); and/or the N-long-chain acyl amino acid dipeptide is N-lauroyl-L-alanyl-L-alanine; and/or the N-long-chain acyl amino acid salt is a lauroyl alanine arginine salt.
[14]. The use according to any one of [1]-[13], characterized in that the composition is prepared as an aqueous solution, an ointment, a liniment, a gel, a cream, a powder, an emulsion, or a spray dosage form; and/or
   the product form corresponding to the composition is a shampoo, a hair conditioner, an anti-hair loss essence, or a hair growth cream.
[15]. The use according to any one of [1]-[14], characterized in that the composition is applied to the hair, optionally massaged, brought into contact with the hair for a certain period of time, and then rinsed off with water.
[16]. A care composition for inducing and/or promoting the growth of keratin fibers and/or slowing down natural loss thereof and/or increasing the density thereof, characterized in that the care composition comprises an N-long-chain acyl amino acid and/or a salt thereof; preferably, the care composition comprises an N-long-chain acyl amino acid and a basic amino acid, or comprises a salt formed from the N-long-chain acyl amino acid and the basic amino acid, or comprises a sodium salt, a potassium salt, or an ammonium salt (e.g., a TEA salt) of the N-long-chain acyl amino acid; further preferably, the N-long-chain acyl amino acid and/or the salt thereof is lauroyl alanine and a basic amino acid, or a salt formed from lauroyl alanine and the basic amino acid, or a sodium salt, a potassium salt, or an ammonium salt of lauroyl alanine.
[17]. Use of a care composition in inducing and/or promoting the growth of keratin fibers and/or slowing down natural loss thereof and/or increasing the density thereof, characterized in that the care composition comprises an N-long-chain acyl amino acid and/or a salt thereof; preferably, the care composition comprises an N-long-chain acyl amino acid and a basic amino acid, or comprises a salt formed from the N-long-chain acyl amino acid and the basic amino acid, or comprises a sodium salt, a potassium salt, or an ammonium salt (e.g., a TEA salt) of the N-long-chain acyl amino acid; further preferably, the care composition comprises lauroyl alanine and a basic amino acid, or a salt formed from lauroyl alanine and the basic amino acid, or a sodium salt, a potassium salt, or an ammonium salt of lauroyl alanine.
[18]. The care composition/use according to [16] or [17], characterized in that the composition further comprises an N-long-chain acyl amino acid dipeptide, or a salt formed from the N-long-chain acyl amino acid dipeptide and a basic amino acid, or a sodium salt, a potassium salt, or an ammonium salt (e.g., a TEA salt) of the N-long-chain acyl amino acid dipeptide; preferably, the care composition further comprises lauroyl alanyl alanine, or a salt formed from lauroyl alanyl alanine and a basic amino acid, or a sodium salt, a potassium salt, or an ammonium salt of lauroyl alanyl alanine.
[19]. The care composition/use according to [16] or [17], characterized in that the N-long-chain acyl amino acid, the basic amino acid, and the N-long-chain acyl amino acid dipeptide are as defined in any one of [7]-[13].
[20]. The care composition according to [16], characterized in that the care composition is prepared as an aqueous solution, an ointment, a liniment, a gel, a cream, a powder, an emulsion, or a spray dosage form; and/or
   the product form corresponding to the care composition is a shampoo, a hair conditioner, an anti-hair loss essence, or a hair growth cream.
[21]. An anti-hair loss shampoo or anti-hair loss essence or hair growth cream, characterized in that the anti-hair loss shampoo or anti-hair loss essence or hair growth cream comprises the care composition according to any one of [16]-[20].
[22]. A non-therapeutic cosmetic method, characterized in that the method comprises topically applying a care composition to the skin or hair of a human or animal; the composition comprises an N-long-chain acyl amino acid and/or a salt thereof; the non-therapeutic cosmetic method serves to activate and moisturize the skin/hair of the human or animal, prevent hair loss, or stimulate hair growth.
[23]. The cosmetic method according to [22], characterized in that the composition comprises an N-long-chain acyl amino acid and a basic amino acid, or comprises a salt formed from the N-long-chain acyl amino acid and the basic amino acid, or comprises a sodium salt, a potassium salt, or an ammonium salt (e.g., a TEA salt) of the N-long-chain acyl amino acid.
[24]. The method according to [22] or [23], characterized in that the composition further comprises an N-long-chain acyl amino acid dipeptide, or a salt formed from the N-long-chain acyl amino acid dipeptide and a basic amino acid, or a sodium salt, a potassium salt, or an ammonium salt (e.g., a TEA salt) of the N-long-chain acyl amino acid dipeptide.
[25]. The method according to any one of [22]-[24], characterized in that the N-long-chain acyl amino acid, the basic amino acid, and the N-long-chain acyl amino acid dipeptide are as defined in any one of [7]-[13].
[26]. The method according to any one of [22]-[25], characterized in that the composition is prepared as an aqueous solution, an ointment, a liniment, a gel, a cream, a powder, an emulsion, or a spray dosage form;
   the product form corresponding to the composition is a shampoo, a hair conditioner, an anti-hair loss essence, or a hair growth cream.
[27]. Use of a composition comprising an N-long-chain acyl amino acid and/or a salt thereof in the manufacture of a medicament for preventing hair loss and/or promoting hair growth and/or increasing hair density.
[28]. The use according to [27], characterized in that the composition comprises an N-long-chain acyl amino acid and a basic amino acid, or comprises a salt formed from the N-long-chain acyl amino acid and the basic amino acid, or comprises a sodium salt, a potassium salt, or an ammonium salt (e.g., a TEA salt) of the N-long-chain acyl amino acid.
[29]. The use according to any one of [27]-[28], characterized in that the composition further comprises an N-long-chain acyl amino acid dipeptide, or a salt formed from the N-long-chain acyl amino acid dipeptide and a basic amino acid, or a sodium salt, a potassium salt, or an ammonium salt (e.g., a TEA salt) of the N-long-chain acyl amino acid dipeptide.
[30]. The use according to any one of [23]-[25], characterized in that the N-long-chain acyl amino acid, the basic amino acid, and the N-long-chain acyl amino acid dipeptide are as defined in any one of [7]-[13].
[31]. The use, the cosmetic method, or the composition according to any one of [1]-[30], characterized in that the percentage by weight of an N-long-chain acyl amino acid and/or a salt thereof in a composition is preferably 1 wt% or greater, further preferably 5 wt% or greater.

Compared with the prior art, the present disclosure has the following beneficial technical effects:
1. In the present disclosure, it is surprisingly found that the composition comprising an N-long-chain acyl amino acid and/or a salt thereof can be used to prevent hair loss and/or promote hair growth and/or increase hair density.
2. The combination of the basic amino acid and the N-long-chain acyl amino acid exhibits an excellent effect on preventing hair loss and/or promoting hair growth and/or increasing hair density.
3. The combination of lauroyl alanine and arginine or the lauroyl alanine arginine salt exhibits a stronger effect on preventing hair loss and/or promoting hair growth and/or increasing hair density than other N-long-chain acyl amino acids and/or salts thereof.

### BRIEF DESCRIPTION OF THE DRAWINGS

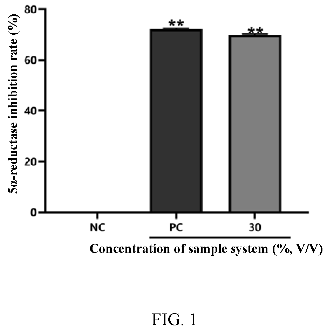

FIG. 1 is a graph showing the 5α-reductase activity inhibition rate of the sample in Example I.

### DETAILED DESCRIPTION

In one embodiment, the present disclosure relates to use of a composition comprising an N-long-chain acyl amino acid and/or a salt thereof in preventing hair loss and/or promoting hair growth and/or increasing hair density, and particularly to cosmetic use.

The term "cosmetic use" is intended to refer to use that is not therapeutic use, pharmaceutical use, or dermatological use; that is, the use does not involve therapeutic treatment and is intended for healthy skin. The term "healthy skin" is intended to refer to skin that is described by experts in the field (e.g., dermatologists) as non-pathological, i.e., not showing any infection, scar, skin disease or skin condition (e.g., candidiasis, impetigo, psoriasis, eczema, acne, or dermatitis), wound or injury, and/or other skin disorders, and/or androgenetic alopecia.

The term "preventing hair loss" refers to protecting against, preventing, inhibiting, or delaying hair loss.

The composition may be a composition for topical administration. The composition may be prepared into an aqueous solution, an ointment, a liniment, a gel, a cream, a powder, an emulsion, a spray, or other dosage forms. The specific product form may be, for example, a shampoo, a hair conditioner, an anti-hair loss essence, or a hair growth cream. For the application method, for example, the composition may be applied to the hair, optionally massaged, brought into contact with the hair for a certain period of time, and then rinsed off with water.

For the N-long-chain acyl amino acid, the N-long-chain acyl in the N-long-chain acyl amino acid is derived from a saturated or unsaturated linear or branched fatty acid with 8-22 carbon atoms. Furthermore, the N-long-chain acyl in the N-long-chain acyl amino acid is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl.

The amino acid in the N-long-chain acyl amino acid is derived from one or more of glycine, alanine, glutamic acid, sarcosine, aspartic acid, leucine, isoleucine, valine, threonine, proline, phenylalanine, arginine, and lysine. Furthermore, the amino acid in the N-long-chain acyl amino acid is derived from one or more of alanine, glycine, glutamic acid, sarcosine, arginine, lysine, and (methyl)taurine, preferably alanine, and most preferably L-alanine.

The N-long-chain acyl(methyl)taurine described in the present disclosure refers to an N-long-chain acyl methyl taurine or an N-long-chain acyl taurine.

As an example, the N-long-chain acyl amino acid may be selected from cocoyl alanine, lauroyl alanine, cocoyl sarcosine, lauroyl sarcosine, lauroyl glutamic acid, cocoyl glutamic acid, oleoyl glutamic acid, cocoyl glycine, lauroyl glycine, stearoyl glutamic acid, and the like, preferably lauroyl alanine and lauroyl sarcosine, and particularly preferably N-lauroyl-L-alanine.

For the synthesis process of N-long-chain acyl amino acids, reference may be made to the general synthesis methods for N-acyl amino acid surfactants, including direct synthesis and indirect synthesis. The direct synthesis methods using fatty acid ingredients include enzymatic synthesis and dehydration synthesis. The indirect synthesis methods include fatty acid chloride acylation, aliphatic nitrile acylation hydrolysis, fatty acid anhydride acylation, amide carbonylation, and the like. One preferred method is the reaction between a fatty acid chloride and the amino group of an amino acid (Schotten-Baumann condensation or Schotten-Baumann reaction).

A typical preparation process suitable for the present disclosure comprises, for example: dissolving an amino acid and sodium hydroxide in water or a mixed solution of water and acetone to give an amino acid salt solution; and slowly and dropwise adding lauroyl chloride and a sodium hydroxide solution to the amino acid salt solution, controlling the pH value of the reaction system, and performing post-treatment on the product after the dropwise addition. Representative methods are disclosed in CN1798821A, US6703517B2, CN102875409B, JPH0570418A, etc.

For the post-treatment of the N-long-chain acyl amino acid product, conventional methods, such as recrystallization, water rinsing, and drying, may be used. One preferred post-treatment comprises: mixing the crude N-long-chain acyl amino acid product with a solvent, optionally stirring, and controlling the temperature T of the mixture system in a range of the melting point of the long-chain fatty acid to the melting point of the N-long-chain acyl amino acid, wherein the solvent is water, an organic solvent, or a mixed solution of water and an organic solvent; and after the temperature of the system is controlled, conducting the solid-liquid separation. Related methods are disclosed by the inventor in CN202210867760.7 and PCT/CN2022/107270, the relevant content of which is incorporated herein.

The N-long-chain acyl amino acid and/or the salt thereof refers to one or a combination of an N-long-chain acyl amino acid and an N-long-chain acyl amino acid salt. The N-long-chain acyl amino acid salt is formed from an N-long-chain acyl amino acid and a base. The base is selected from one or more of an inorganic base, an organic amine, and a basic amino acid; the inorganic base is selected from one or more of sodium hydroxide, potassium hydroxide, sodium carbonate, and potassium carbonate, preferably sodium hydroxide and potassium hydroxide; the organic amine is selected from amine, alkanolamine, and the like.

The basic amino acid is selected from one or more of arginine, lysine, citrulline, ornithine, creatine, histidine, diaminobutyric acid, and diaminopropionic acid, preferably arginine and/or lysine, and most preferably L-arginine.

In addition, the composition may further comprise an N-long-chain acyl amino acid dipeptide, or a salt formed from an N-long-chain acyl amino acid dipeptide and a basic amino acid, or a sodium salt, a potassium salt, or an ammonium salt of the N-long-chain acyl amino acid dipeptide.

The N-long-chain acyl amino acid dipeptide is a known concept, and refers to a long-chain acyl aminoacyl amino acid, typically, for example, long-chain acyl glycyl glycine, long-chain acyl glutamyl glutamic acid, long-chain acyl alanyl alanine, or long-chain acyl sarcosyl sarcosine. See, e.g., CN100448968C and PCT/CN2022/107270.

The N-long-chain acyl amino acid dipeptide may be prepared from an acid chloride derived from an N-long-chain acyl amino acid and an amino acid by the Schotten-Baumann reaction or the like, or by the reaction of an amino acid dipeptide (such as glutamyl glutamic acid) and an acid chloride. One preferred relevant preparation method is disclosed in PCT/CN2022/107270, the relevant content of which is incorporated herein.

The N-long-chain acyl in the N-long-chain acyl amino acid dipeptide is selected from one or more of octanoyl, caprinoyl, undecanoyl, lauroyl, myristoyl, pentadecanoyl, palmitoyl, stearoyl, oleoyl, linoleoyl, isostearoyl, coconut oil fatty acyl, and palm oil fatty acyl, preferably coconut oil fatty acyl and lauroyl, and most preferably lauroyl.

The amino acid in the N-long-chain acyl amino acid dipeptide is derived from one or more of alanine, glycine, glutamic acid, sarcosine, arginine, lysine, and (methyl)taurine, preferably L-alanine. An N-long-chain acyl-L-alanine dipeptide, particularly, N-lauroyl-L-alanyl-L-alanine, is preferred in the present disclosure.

Preferably, the basic amino acid is present in an amount fully or partially neutralizing the N-long-chain acyl amino acid, preferably with the degree of neutralization being 85% or greater and less than 100%, more preferably 88% or greater and 96% or less; or

the basic amino acid is present in an amount fully or partially neutralizing the N-long-chain acyl amino acid or the N-long-chain acyl amino acid dipeptide, preferably with the degree of neutralization being 85% or greater and less than 100%, more preferably 88% or greater and 96% or less.

In one embodiment, the present disclosure relates to a care composition for inducing and/or promoting the growth of keratin fibers and/or slowing down natural loss thereof and/or increasing the density thereof. Certainly, the present disclosure also relates to use of a care composition in inducing and/or promoting the growth of keratin fibers and/or slowing down natural loss thereof and/or increasing the density thereof.

The care composition comprises an N-long-chain acyl amino acid and/or a salt thereof. Preferably, the N-long-chain acyl amino acid and/or the salt thereof are/is a primary surfactant in the care composition. The primary surfactant means that in terms of the percentage by weight, the surfactant has the highest content among all the surfactants. Preferably, the percentage by weight of the primary surfactant in all the surfactants is greater than 50 wt%, for example, greater than 55 wt%, 60 wt%, 65 wt%, 70 wt%, 75 wt%, or 80 wt%. In addition to the primary surfactant, other surfactants are co-surfactants. The co-surfactant means that compared with the primary surfactant, the amount of the co-surfactant used is less than that of the primary surfactant, playing an auxiliary role in cleaning. The co-surfactant is not essential. Certainly, in most applications, it is preferred to add a small amount of co-surfactant.

The care composition comprises an N-long-chain acyl amino acid and a basic amino acid, or comprises a salt formed from the N-long-chain acyl amino acid and the basic amino acid, or comprises a sodium salt, a potassium salt, or an ammonium salt of the N-long-chain acyl amino acid.

The term "keratin fiber" refers to hair, eyebrows, and/or eyelashes. Preferably, the keratin fiber is hair.

The care composition may further comprise an N-long-chain acyl amino acid dipeptide, or a salt formed from the N-long-chain acyl amino acid dipeptide and a basic amino acid, or a sodium salt, a potassium salt, or an ammonium salt of the N-long-chain acyl amino acid dipeptide.

For the N-long-chain acyl amino acid, the basic amino acid, and the N-long-chain acyl amino acid dipeptide, reference is made to the definitions and preferred types in the embodiments described above, and details are not described herein again.

The care composition may be a composition for topical administration. The composition may be prepared into an aqueous solution, an ointment, a liniment, a gel, a cream, a powder, an emulsion, a spray, or other dosage forms. The specific product form may be, for example, a shampoo, a hair conditioner, an anti-hair loss essence, or a hair growth cream. For the application method, for example, the composition may be applied to the hair, optionally massaged, brought into contact with the hair for a certain period of time, and then rinsed off with water.

In one embodiment, the present disclosure relates to a non-therapeutic cosmetic method, which comprises topically applying a care composition to the skin or hair of a human or animal, wherein the composition comprises an N-long-chain acyl amino acid and/or a salt thereof; the non-therapeutic cosmetic method serves to activate and moisturize the skin/hair of the human or animal, prevent hair loss, or stimulate hair growth. Preferably, the non-therapeutic cosmetic method is a cosmetic method for protecting against, preventing, inhibiting, or delaying hair loss.

Furthermore, the composition comprises an N-long-chain acyl amino acid and a basic amino acid, or comprises a salt formed from the N-long-chain acyl amino acid and the basic amino acid, or comprises a sodium salt, a potassium salt, or an ammonium salt of the N-long-chain acyl amino acid.

The composition may further comprise an N-long-chain acyl amino acid dipeptide, or a salt formed from the N-long-chain acyl amino acid dipeptide and a basic amino acid, or a sodium salt, a potassium salt, or an ammonium salt of the N-long-chain acyl amino acid dipeptide.

For the N-long-chain acyl amino acid, the basic amino acid, and the N-long-chain acyl amino acid dipeptide, reference is made to the definitions and preferred types in the embodiments described above, and details are not described herein again.

The composition may be a composition for topical administration. The composition may be prepared into an aqueous solution, an ointment, a liniment, a gel, a cream, a powder, an emulsion, a spray, or other dosage forms. The specific product form may be, for example, a shampoo, a hair conditioner, an anti-hair loss essence, or a hair growth cream. For the application method, for example, the composition may be applied to the hair, optionally massaged, brought into contact with the hair for a certain period of time, and then rinsed off with water.

In one embodiment, the present disclosure relates to use of a composition comprising an N-long-chain acyl amino acid and/or a salt thereof in the manufacture of a medicament for preventing hair loss and/or promoting hair growth and/or increasing hair density.

Furthermore, the composition comprises an N-long-chain acyl amino acid and a basic amino acid, or comprises a salt formed from the N-long-chain acyl amino acid and the basic amino acid, or comprises a sodium salt, a potassium salt, or an ammonium salt of the N-long-chain acyl amino acid.

The composition may further comprise an N-long-chain acyl amino acid dipeptide, or a salt formed from the N-long-chain acyl amino acid dipeptide and a basic amino acid, or a sodium salt, a potassium salt, or an ammonium salt of the N-long-chain acyl amino acid dipeptide.

For the N-long-chain acyl amino acid, the basic amino acid, and the N-long-chain acyl amino acid dipeptide, reference is made to the definitions and preferred types in the embodiments described above, and details are not described herein again.

In the above embodiments, the content of the N-long-chain acyl amino acid and/or the salt thereof in the composition is preferably 1 wt% or greater, more preferably 5 wt% or greater, for example, 1 wt%, 2 wt%, 3 wt%, 4 wt%, 5 wt%, 6 wt%, 7 wt%, 8 wt%, 9 wt%, or 10 wt% or greater.

In the above embodiments, the composition of the present disclosure may optionally further comprise other ingredients, such as essences and fragrances, dyes, surfactants, thickening agents, humectants, chelating agents, extracts, amino acids, nucleic acids, vitamins, enzymes, antiinflammatory agents, bactericides, antioxidants, ultraviolet absorbers, antiperspirants, pH regulators, and pearlizing agents.

In the embodiments, wherever the details, type selection, amount selection, and the like that are not discussed do not conflict with the content, reference may be made to the related teachings in other embodiments, which will not be recited for brevity.

The present disclosure will be further illustrated with reference to the following drawings and examples. It will be appreciated that these examples are merely intended to illustrate the present disclosure rather than limit the scope of the present disclosure. Furthermore, it will be appreciated that various changes or modifications of the present disclosure can be made by those skilled in the art after reading the teachings of the present disclosure, and such equivalents also fall within the scope of the appended claims of the present application.

### Examples

### 1 Test objective

Androgenetic alopecia (AGA) is caused by the conversion of testosterone (T) into more potent dihydrotestosterone (DHT) under the action of 5α-reductase. Hair follicles are sensitive to DHT, and this affects the development of hair follicles and thereby leads to hair loss. This experiment analyzes whether the sample can inhibit 5α-reductase and thus inhibit the generation of DHT, which can be used as the basis for determining whether the sample has an anti-hair loss effect.

### 2 Test item

Anti-hair loss efficacy test - 5α-reductase inhibition rate.

### 3 Test materials

### 3.1 Main reagents

1) Type II 5α-reductase: extracted from rat prostate tissue, preserved at -80 °C.
2) Positive control (PC): 1 µM finasteride solution.
3) Blank control (BC): phosphate buffer (PBS).

### 3.2 Main instrument

High-performance liquid chromatograph (HPLC).

### 4 Test methods

4.1 Preparation of type II 5α-reductase: 5 male SD rats were fasted but had free access to water overnight, and then sacrificed by asphyxiation with CO₂. The prostate was taken out and cut into pieces on an ice platform. The sample was homogenized in a glass homogenizer with precooled buffer at 0 °C in a ratio of 1:5 and centrifuged at 3000 g for 10 min. The supernatant was centrifuged at 10,000 g for 30 min and aliquoted into EP tubes at 1 mL per tube to give an enzyme extract, which was preserved in a freezer at -80 °C.

4.2 Determination of type II 5α-reductase activity: A 2 mL reaction system contained 0.25 mL of a phosphate buffer, 0.70 mL of the enzyme extract, 0.1 mL of a testosterone solution (0.5 mg/mL), 0.35 mL of a NADPH solution (1 mg/mL), and 0.6 mL of (10% ethanol for the complete reaction group, finasteride for the positive control group, and the sample solution for the sample group). The enzyme extract was replaced with a phosphate buffer in the control tube, and 2.0 mL of dichloromethane was added to terminate the reaction. The reaction in the sample tube was conducted at 37 °C for 30 min. After the reaction was completed, 2.0 mL of dichloromethane was added to stop the reaction. The mixture was shaken for 1 min and centrifuged at 5000 r/min for 10 min. The upper aqueous phase was discarded. About 1.0 mL of the organic phase was taken and concentrated by evaporation, and the residue was dissolved in 1.0 mL of methanol. The peak area of testosterone (T) was determined by high-performance liquid chromatography. 3 replicate tubes were set for each reaction. Calculation of inhibition rate: $Inhibition rate % = \frac{{T}_{sample tube} - {T}_{reaction tube}}{{T}_{blank tube} - {T}_{reaction tube}} \times 100 %$

### 5 Test results

### I. Results of experiments conducted at SINOLIGHT SLSAC

Sample preparation (TA): lauroyl alanine 6% + arginine 3.84% + benzoic acid 0.2% + essence 0.25% + balance of water (TO 100). The experimental system was then diluted with a PBS buffer to give a sample at a reaction concentration of 30% (V/V).

The results of the 5α-reductase activity inhibition rate are shown in Table 1 and FIG. 1.

**Table 1. Summary of results of 5α-reductase activity inhibition rate**

| **Group** | **Concentration of system (%, V/V)** | **5α-reductase activity inhibition rate (%)** | **SD** | ***p*-value** |
|---|---|---|---|---|
| Blank control (BC) | / | / | / | / |
| Negative control (NC) | / | 0.00 | 0.50 | / |
| Positive control (PC) | 1 | 72.21 | 0.37 | 0.005** |
| Sample | 30 | 69.77 | 0.43 | 0.002** |

| | | | | |
|---|---|---|---|---|
| Note: When the statistical analysis is performed by using the *t*-test method, the sample group and the PC group are compared with the complete reaction group, and the significance is indicated by *. * denotes *p*-value < 0.05, and ** denotes *p*-value < 0.01. | | | | |

Compared with the NC group, at an administration concentration of 1 µM, the 5α-reductase inhibition rate of the PC group was 72.21%, with a statistical difference (p < 0.01), indicating that the positive control test was valid.

Compared with the NC group, at a system concentration of 30% (V/V), the 5α-reductase inhibition rate of the sample was 69.77%, with a statistical difference (p < 0.01).

### II. Results of experiments conducted at Guangzhou Chn-Alternative Biotechnology Co., Ltd.

### Positive control 1: finasteride

0.0372 g of finasteride was weighed out and diluted to 1 mL with absolute ethanol. 0.005 mL of the mixture was taken, and 4.995 mL of absolute ethanol was added to give a stock solution. Another 0.005 mL of the mixture was then taken, and 0.495 mL of absolute ethanol was added to prepare a finasteride solution at a concentration of 1 µmol/L.

### Positive control 2: minoxidil

0.50 g of minoxidil was weighed out, 1 g of propylene glycol was added, and 8.50 g of 75% alcohol was added.

### Sample preparation:

(1) 83.90 g of water was first added, and then 10 g of lauroyl alanine was added. The temperature was raised to 80-85 °C and maintained at this temperature. 6.10 g of arginine was then added, and the mixture was stirred for dissolution to give a sample of lauroyl alanine arginine salt, LA-Arg (10%) (the content of lauroyl alanine in the sample being 10 wt% based on lauroyl alanine). The above sample was diluted with a PBS buffer to give a sample with a lauroyl alanine content of 5 wt%, LA-Arg (5%).
(2) 88.6 g of ultrapure water was first added, and then 1.4 g of sodium hydroxide was added. The temperature was raised to 80-85 °C and maintained after dissolution. 10 g of lauroyl alanine was then added, and the mixture was stirred for dissolution to give a sample of sodium lauroyl alaninate, LA-Na (10%) (the content of lauroyl alanine in the sample being 10 wt% based on lauroyl alanine). The above sample was diluted with a PBS buffer to give a sample with a lauroyl alanine content of 5 wt%, LA-Na (5%).
(3) 90 g of water was first added, and then 10 g of arginine was added to give sample Arg (10%) (the content of arginine in the sample being 10 wt%). The above sample was diluted with a PBS buffer to give a sample with an arginine content of 5 wt%, Arg (5%).
(4) 84.76 g of water was first added, and then 10 g of lauroyl glutamic acid was added. The temperature was raised to 80-85 °C and maintained at this temperature. 5.24 g of arginine was then added, and the mixture was stirred for dissolution to give a sample of lauroyl glutamic acid arginine salt, Glu-Arg (10%) (the content of lauroyl glutamic acid in the sample being 10 wt%). The above sample was diluted with a PBS buffer to give a sample with a lauroyl glutamic acid content of 5 wt%, Glu-Arg (5%).
(5) 83.23 g of water was first added, and then 10 g of lauroyl glycine was added. The temperature was raised to 80-85 °C and maintained at this temperature. 6.77 g of arginine was then added, and the mixture was stirred for dissolution to give a sample of lauroyl glycine arginine salt, Gly-Arg (10%) (the content of lauroyl glycine in the sample being 10 wt%). The above sample was diluted with a PBS buffer to give a sample with a lauroyl glycine content of 5 wt%, Gly-Arg (5%).

The results of the 5α-reductase activity inhibition rate are shown in Table 2.

**Table 2. Summary of results of 5α-reductase activity inhibition rate**

| **Group** | **5α-reductase activity inhibition rate (%)** |
|---|---|
| Blank control group | / |
| Complete reaction group | 0.00±0.51 |
| Positive control 1: finasteride | 79.79±0.33* |
| Positive control 2: minoxidil | 17.82±0.24* |
| Sample LA-Arg (10%) | 73.24±0.26* |
| Sample LA-Arg (5%) | 90.06±0.34* |
| Sample LA-Na (10%) | 67.26±0.83* |
| Sample LA-Na (5%) | 48.69±0.37* |
| Sample Arg (10%) | 94.08±0.41* |
| Sample Arg (5%) | 18.83±0.44* |
| Sample Glu-Arg (10%) | 49.65±0.15* |
| Sample Glu-Arg (5%) | 42.39±0.13* |
| Sample Gly-Arg (10%) | 56.89±0.56* |
| Sample Gly-Arg (5%) | 48.97±0.14* |

| | |
|---|---|
| *: Compared with the complete reaction group, the difference is statistically significant (P < 0.05). | |

Moreover, those skilled in the art will appreciate that although some embodiments described herein include some features included in other embodiments, the combinations of features in different embodiments shall fall within the scope of the present disclosure and form different embodiments. For example, in the claims, any one of the claimed embodiments may be used in arbitrary combinations.

## Claims

1. Use of a composition comprising an N-long-chain acyl amino acid and/or a salt thereof in preventing hair loss and/or promoting hair growth and/or increasing hair density.

2. The use according to claim 1, **characterized in that** the use is cosmetic use; and/or the composition is a composition for topical administration.

3. The use according to claim 1 or 2, **characterized in that** the composition comprises an N-long-chain acyl amino acid and a basic amino acid, or comprises a salt formed from the N-long-chain acyl amino acid and the basic amino acid, or comprises a sodium salt, a potassium salt, or an ammonium salt of the N-long-chain acyl amino acid.

4. The use according to any one of claims 1-3, **characterized in that** the composition further comprises an N-long-chain acyl amino acid dipeptide and/or a salt thereof.

5. The use according to any one of claims 1-4, **characterized in that** the N-long-chain acyl in the N-long-chain acyl amino acid is derived from a saturated or unsaturated linear or branched fatty acid with 8-22 carbon atoms; preferably, the N-long-chain acyl is selected from cocoyl and lauroyl, most preferably lauroyl; and/or
the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, and (methyl)taurine, preferably alanine, and most preferably L-alanine.

6. The use according to claim 3, **characterized in that** the basic amino acid is selected from one or more of arginine, lysine, citrulline, ornithine, creatine, histidine, diaminobutyric acid, and diaminopropionic acid, preferably arginine and/or lysine, and most preferably L-arginine.

7. The use according to any one of claims 1-6, **characterized in that** the composition comprises lauroyl alanine and arginine, or comprises a lauroyl alanine arginine salt.

8. A care composition for inducing and/or promoting the growth of keratin fibers and/or slowing down natural loss thereof and/or increasing the density thereof, **characterized in that** the care composition comprises an N-long-chain acyl amino acid and/or a salt thereof; the N-long-chain acyl amino acid and/or the salt thereof are/is lauroyl alanine and a basic amino acid, or a salt formed from lauroyl alanine and the basic amino acid, or a sodium salt, a potassium salt, or an ammonium salt of lauroyl alanine.

9. The care composition according to claim 8, **characterized in that** the care composition further comprises lauroyl alanyl alanine, or a salt formed from lauroyl alanyl alanine and a basic amino acid, or a sodium salt, a potassium salt, or an ammonium salt of lauroyl alanyl alanine.

10. The care composition according to claim 8 or 9, **characterized in that** the basic amino acid is selected from one or more of arginine, lysine, citrulline, ornithine, creatine, histidine, diaminobutyric acid, and diaminopropionic acid, preferably arginine and/or lysine, and most preferably L-arginine.

11. The care composition according to claim 8, **characterized in that** the N-long-chain acyl amino acid and/or the salt thereof are/is a primary surfactant in the care composition; preferably, the percentage by weight of the lauroyl alanine and/or the salt thereof in all surfactants is greater than 60 wt%, more preferably greater than 70 wt%.

12. An anti-hair loss shampoo or anti-hair loss essence or hair growth cream, **characterized in that** the anti-hair loss shampoo or anti-hair loss essence or hair growth cream comprises the care composition according to any one of claims 8-11.

13. A non-therapeutic cosmetic method, **characterized in that** the method comprises topically applying a care composition to the skin or hair of a human or animal; the care composition comprises an N-long-chain acyl amino acid and/or a salt thereof; the non-therapeutic cosmetic method serves to activate and moisturize the skin/hair of the human or animal, prevent hair loss, or stimulate hair growth.

14. The cosmetic method according to claim 13, **characterized in that** the care composition comprises an N-long-chain acyl amino acid and a basic amino acid, or comprises a salt formed from the N-long-chain acyl amino acid and the basic amino acid, or comprises a sodium salt, a potassium salt, or an ammonium salt of the N-long-chain acyl amino acid.

15. The cosmetic method according to claim 13, **characterized in that** the care composition further comprises an N-long-chain acyl amino acid dipeptide and/or a salt thereof.

16. The cosmetic method according to any one of claims 13-15, **characterized in that**
the N-long-chain acyl in the N-long-chain acyl amino acid is derived from a saturated or unsaturated linear or branched fatty acid with 8-22 carbon atoms; preferably, the N-long-chain acyl is selected from cocoyl and lauroyl, most preferably lauroyl; and/or
the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, and (methyl)taurine, preferably alanine, and most preferably L-alanine.

17. The cosmetic method according to claim 14, **characterized in that** the basic amino acid is selected from one or more of arginine, lysine, citrulline, ornithine, creatine, histidine, diaminobutyric acid, and diaminopropionic acid, preferably arginine and/or lysine, and most preferably L-arginine.

18. Use of a composition comprising an N-long-chain acyl amino acid and/or a salt thereof in the manufacture of a medicament for preventing hair loss and/or promoting hair growth and/or increasing hair density.

19. The use according to claim 18, **characterized in that** the composition comprises an N-long-chain acyl amino acid and a basic amino acid, or comprises a salt formed from the N-long-chain acyl amino acid and the basic amino acid, or comprises a sodium salt, a potassium salt, or an ammonium salt of the N-long-chain acyl amino acid.

20. The use according to claim 18, **characterized in that** the composition further comprises an N-long-chain acyl amino acid dipeptide and/or a salt thereof.

21. The use according to any one of claims 18-20, **characterized in that** the N-long-chain acyl in the N-long-chain acyl amino acid is derived from a saturated or unsaturated linear or branched fatty acid with 8-22 carbon atoms; preferably, the N-long-chain acyl is selected from cocoyl and lauroyl, most preferably lauroyl; and/or
the amino acid in the N-long-chain acyl amino acid is derived from one or more of glycine, alanine, glutamic acid, sarcosine, aspartic acid, leucine, isoleucine, valine, threonine, proline, phenylalanine, arginine, lysine, and (methyl)taurine, preferably alanine, and most preferably L-alanine.

22. The use according to claim 19, **characterized in that** the basic amino acid is selected from one or more of arginine, lysine, citrulline, ornithine, creatine, histidine, diaminobutyric acid, and diaminopropionic acid, preferably arginine and/or lysine, and most preferably L-arginine.
